# EUROPEAN PATENT SPECIFICATION

(11) **EP 1 023 041 B1**
(45) Date of publication and mention of the grant of the patent: **19.01.2005**
(21) Application number: 98954343.4
(22) Date of filing: 06.10.1998
(51) Int. Cl.: A61K 7/48, A61K 7/00

(54) **CO-GROUND PRODUCTS FOR THE COSMETIC AND DERMATOLOGICAL USE**
COVERMAHLENE PRODUKTE FÜR DERMATOLOGISCHE UND KOSMETISCHE ZWECKE
PRODUITS A USAGE COSMETIQUE ET DERMATOLOGIQUE OBTENUS PAR COBROYAGE

(30) Priority: 07.10.1997 IT MI972276
(43) Date of publication of application: 02.08.2000
(73) Proprietor: Telos S.R.L., 34100 Trieste (IT)
(72) Inventor: BADER, Stefano, I-34100 Trieste (IT); MASIELLO, Sandra, I-34100 Trieste (IT)
(74) Representative: Minoja, Fabrizio, Dr.
(86) International application number: PCT/EP1998/006329
(87) International publication number: WO 1999/017736

(56) References cited:
- EP-A- 0 154 679
- EP-A- 0 449 167
- WO-A-96/32931

## Description

The present invention relates to a process for the preparation of co-ground products (hereinafter "Co-grounds") for the cosmetic and dermatological use.

The co-grinding technique has already been used in the pharmaceutical field in order to increase the dissolution rate of active ingredients (hereinbelow "Actives") (EP 371 431).

For this purpose, the active ingredient is co-ground with a carrier in the presence of a suitable solvent vapour, so as to obtain the partial or total amorphization of the Active (originally in the crystalline form).

Amorphization results in a much higher solubilisation kinetics profile, in conditions of supersaturation, than when using the Active in the crystalline form.

A further object is also to improve the wettability characteristics of the pharmaceutical Active and its dissolution rate.

In order to achieve the desired results, co-grinding has to be carried out using high-energy mills.

This technique has been applied in the preparation of Co-grounds mainly intended for pharmaceuticals to be administered orally, in order to overcome the problems related to the poor solubility of the active ingredient.

WO 99/17736 discloses mechanically activated composites of sodium starch glycolate and pharmaceutically active substances having enhanced dissolution rates and solubilisation kinetics of the active substances when compared to the active substance per se. Further to improved bioavailability, the composites also mask the strong taste of some active principles. These composites can be prepared by mixing together in a solid mixer an active substance and sodium starch glycolate, both in the powder form. It is also stated that the process can be carried out even without adding solvents or solvent vapours, but this possibility seem strictly related to the presence of starch glycolate, which is a hydrophylic polymer and contains some water which might be released throughout the process. Moreover, cosmetic actives and compositions are not mentioned in the application and the examples refer only to oral pharmaceutical forms, namely tablets and capsules.

The co-grinding technique in the cosmetic field has been disclosed in EP 0154679, which relates to high quality nail polish compositions and to a process for preparing them. The process requires the use of solvents and affords a pigment paste that is subsequently combined and mixed with a lacquer formulation. In fact, the first step of the process consists in grinding a plurality of individual pigments in a mill base grinding medium comprising a water-insoluble protective colloid and a low volatizing plasticizer in amounts sufficient to form a pigment dispersing paste.

Thus, there is still the need to develop a process for the preparation of co-grounds intended for cosmetic and dermatological use.

It has now been found that such co-grounds can be obtained through the technique described in the following, which provide the following advantages:
- Increase in the stability of both the Active and the cosmetic formulation.
- Increase in the bio-availability of Actives and consequent improved effectiveness of the cosmetic formulations.
- Increase in the safety and harmlessness of a finished product.
- Availability of controlled-release and modular-release systems.
- Increase in the applicative sensory characteristics of finished products.

The process of the invention comprises co-grinding, without the use of solvents, (i) at least a cosmetic active and (ii) a carrier able to modify the hydrophilic or lipophilic characteristics of the system or its release or absorption characteristics, and/or inert inorganic materials, in the homogeneously split crystalline on amorphous forms.

It is preferable to use two components, although suitable Co-grounds for use in cosmetics can be obtained from ternary and/or higher mixtures.

Usually, according to the invention, a cosmetic Active is co-ground with a carrier, but co-grinding two actives or two carriers together is also possible, when the object is to modify the chemical, physical or sensory characteristics.

The cosmetic active is selected from hydrating agents, nutrients, emollients, soothing agents, sun protection agents, anti-ageing agents, de-pigmentation agents, depilatory agents.

The active can be solid or rendered solid via absorption, inclusion or micro-incapsulation techniques.

"Actives" also mean dyes, pigments, flavours or fragrances.

Suitable carriers comprise hydrophilic or lipophilic polymers, such as crospovidone, pectin, dextran, polyvinylpyrrolidone, cellulose and its derivatives, starches and their derivatives; cyclodextrins; phospholipids; fatty acids; sugars.

Examples of inert inorganic materials comprise talc, titanium dioxide, zinc oxide, kaolin, zeolites, SiO₂, silica gel, calcium phosphate, calcium carbonate and the like.

In view of what stated above, according to the intended purposes, a component of the Co-ground may be considered as both an Active and a carrier or an inert material, depending on the cases. For example, titanium dioxide can be considered both as a pigment having a cosmetic function or as an inert carrier.

The weight ratios of active ingredient to inert carrier, in the case of binary Co-grounds, can range within wide limits, but they will generally range from 1:10 to 10:1.

The co-grinding process is carried out in a conventional apparatus, such as a ball mill, a cylinder mill, a rotary mill, a grinding mill or a vibrational mill, for times ranging from a few minutes to some hours, for example from 30' to 8 hours.

Preferably, the Co-grinding process is carried out in a mill which transfers vibrational energy to the Active / Support mix.

This apparatus has the special characteristic of using small amounts of energy at a high frequency of vibration.

In fact, only small impactual forces are necessary for the Co-grinding process. Any excess force would result in a waste of energy dissipated as heat.

The mill is composed of a cylindrical chamber in stainless steel with a polyurethane covering. Small highly packed grinding media made from a very hard material are placed inside the chamber.

The choice of the size and material of the grinding media depends on the properties of the material to be processed and the desired characteristics of the finished product.

The variation in some operating parameters results in a different energy transfer from the mill to the mixture and thus in different final characteristics of the product.

The vibratory mechanism is made up of a special electric motor linked to two "out of balance" counterweights. This entire group of components, attached directly to the base of the grinding chamber, is suspended by high-tension steel springs in order that energy is directly imparted to the grinding media.

The vibration created by the movement of this system is defined according to two physics-related measures: frequency and amplitude. The particularity lies in the fact that the vibration is of a tridimensional type, in that it is characterized by a horizontal as well as a vertical component. The former can be modified by changing the grinding angle i.e. by varying the position of one of the two counterweights. In this way a different load movement is attained, and in addition the energy transmitted to the grinding media is regulated.

The effect of the Co-grinding process in a vibrational mill is determined by a number of parameters which may be adjusted by the skilled person according to the desired objectives. Said parameters include the fill-level of the chamber, in addition to the shape, volume and density of the grinding media.

Another important factor in determining the specific energy used in the Co-grinding process is the ratio between the mass of material to be ground and the mass of the grinding media. If the ratio is highly skewed towards the grinding media then the energy will be higher.

The correct duration of the process clearly depends on the chemical and physical characteristics of the materials to be Co-ground, as well as on the above-described factors.

Finally, the choice of the weight ratio between the Active and the Carrier logically depends on the chemical and physical characteristics of the starting materials, and on the desired final objectives.

The co-grounds obtained according to the invention can be used as they are or in a mixture with suitable excipients for the preparation of cosmetic formulations having advantageous characteristics.

More particularly, Actives co-ground with suitable carriers have improved characteristics of tolerability, stability, solubility and compatibility with other cosmetic ingredients.

The following examples illustrate the invention in greater detail.

### Example 1

3.3 grams of gingko biloba dry extract and 16.5 grams of pectin are sieved through a 250 µm (60 mesh) sieve and mixed for 5 minutes. The mixture is placed in the grinding chamber of a vibrational mill together with conventional grinding media.

Co-grinding is carried out for 4 hours.

### Example 2

2 grams of 18,β-glycyrrhetinic acid and 18 grams of β-cyclodextrin are sieved through a 250 µm (60 mesh) sieve and mixed for 5 minutes. The mixture is placed in the grinding chamber together with the grinding media, as in Example 1.

Co-grinding is carried out for 6 hours.

### Example 3

2 grams of 18,β-glycyrrhetinic acid and 18 grams of micronized crospovidone are sieved through a 250 µm (60 mesh) sieve and mixed for 5 minutes. The mixture is placed in the grinding chamber together with the grinding media, as in Example 1.

Co-grinding is carried out for 6 hours.

### Example 4

5 grams of butyl methoxydibenzoylmethane and 15 grams of micronized crospovidone are sieved through a 250 µm (60 mesh) sieve and mixed for 5 minutes. The mixture is placed in the grinding chamber together with the grinding media, as in Example 1.

Co-grinding is carried out for 6 hours.

### Example 5

5 grams of aloe and 15 grams of pectin are sieved through a 250 µm (60 mesh) sieve and mixed for 5 minutes. The mixture is placed in the grinding chamber together with the grinding media, as in Example 1.

Co-grinding is carried out for 4 hours.

### Example 6

6.7 grams of 18,β-glycyrrhetinic acid and 13.4 grams of hydrogenated soy lecithin are sieved through a 250 µm (60 mesh) sieve and mixed for 5 minutes. The mixture is placed in the grinding chamber together with the grinding media and the mass is cooled at -18°C for 10 minutes.

Co-grinding is carried out for 1 hour.

The products obtained according to Examples 1, 4, 5 and 6 were tested for their dissolution rates.

The results, compared with the values obtained with the simple physical mixture of the components, proved that this parameter can be modified, i.e. increased or decreased, depending on the characteristics of the selected carrier (Tables 1, 4, 5 and 6).

For all the tested Actives, the method described in Italian Pharmacopoeia IX Ed. was followed, using a Sotax apparatus temperature-controlled at 32°C and a DU 65 Beckman spectrophotometer.

In all cases the sample amounts used were such as to ensure that sink conditions, i.e. concentrations below 20% of the solubility, were maintained.

For products containing Aloe, 900 ml of acetate buffer solution (pH 5.5) mixed at 50 rpm were used; wavelength was set at 295 nm.

For products containing gingko biloba, 900 ml of acetate buffer solution (pH 5.5) mixed at 50 rpm were used; wavelength was set at 262 nm.

For products containing butyl methoxydibenzoylmethane, 900 ml of a 0.5% solution of SLS (sodium lauryl sulfate) in acetate buffer (pH 5.5) mixed at 50 rpm were used; wavelength was set at 262 nm.

For products containing 18,β-glycyrrhetinic acid, 900 ml of an ethanol/deionized water 10/90 solution mixed at 100 rpm were used; wavelength was set at 257 nm.

**Table 1**

| Gingko biloba concentration (µg/ml). | | |
|---|---|---|
| Time | Physical mixture | Co-ground |
| 2 min | 58.02 | 9.39 |
| 5 min | 58.27 | 23.72 |
| 10 min | 57.54 | 36.11 |
| 20 min | 58.38 | 48.78 |
| 30 min | 57.72 | 53.64 |
| 60 min | - | 57.24 |

**Table 2**

| 18,β-glycyrrhetinic acid concentration (µg/ml). | | |
|---|---|---|
| Time | Physical mixture | Co-ground |
| 30 min | 1.63 | 18.12 |

**Table 3**

| 18,β-glycyrrhetinic acid concentration (µg/ml). | | |
|---|---|---|
| Time | Physical mixture | Co-ground |
| 30 min | 1.63 | 10.76 |

**Table 4**

| Butyl methoxydibenzoylmethane concentration (µg/ml). | | |
|---|---|---|
| Time | Physical mixture | Co-ground |
| 2 min | 0.14 | 7.62 |
| 5 min | 0.26 | 11.75 |
| 10 min | 0.44 | 13.09 |
| 20 min | 0.94 | 13.07 |
| 30 min | 1.43 | 12.92 |
| 60 min | 2.86 | - |

**Table 5**

| Aloe concentration (µg/ml). | | |
|---|---|---|
| Time | Physical mixture | Co-ground |
| 2 min | 54.62 | 8.21 |
| 5 min | 57.05 | 14.35 |
| 10 min | 57.36 | 23.15 |
| 20 min | 57.68 | 32.55 |
| 30 min | 57.35 | 39.75 |
| 60 min | 57.38 | 52.36 |

**Table 6**

| 18,β-glycyrrhetinic acid concentration (µg/ml). | | |
|---|---|---|
| Time | Physical mixture | Co-ground |
| 1 min | 2.49 | 7.63 |
| 5 min | 4.89 | 11.78 |
| 15 min | 4.04 | 16.14 |
| 30 min | 5.55 | 18.61 |
| 60 min | 5.96 | 21.06 |

The products obtained following the examples 2 and 3 were tested for their solubilisation kinetics, under non-sink conditions, i.e. with the Active in excess to its solubility. The co-ground products were placed in flasks with about 50 ml of acetate buffer solution (pH 5.5), then placed in a stirrer temperature-controlled at 32°C and stirred at 50 rpm.

At predetermined times, aliquots of the solution were withdrawn, filtered and analyzed using a spectrophotometer at 254 nm wavelength.

As it can be seen in tables 2 and 3, values increase remarkably for co-ground products.

## Claims

1. A process for the preparation of co-grounds for the cosmetic and dermatological use, comprising co-grinding, without the use of solvents, (i) at least a cosmetic active and **(ii)** a carrier able to modify the hydrophilic or lipophilic characteristics of the system or its release or absorption characteristics, and/or inert inorganic materials, in the homogeneously split crystalline or amorphous forms, said cosmetic active being selected from hydrating agents, nutrients, emollients, soothing agents, sun protection agents, anti-ageing agents, de-pigmentation agents, depilatory agents, dyes, pigments, flavours or fragrances.

2. A process as claimed in claim 1, in which an active ingredient is co-grinded with a carrier.

3. A process as claimed in claim 2, in which the actives are solid or have been rendered solid via absorption, inclusion or micro-incapsulation techniques.

4. A process as claimed in claim 1 or 2, in which the carriers are hydrophilic or lipophilic polymers, selected from crospovidone, pectin, dextran, polyvinylpyrrolidone, cellulose and its derivatives, starches and their derivatives; cyclodextrins; phospholipids; fatty acids; sugars.

5. A process as claimed in claim 1, wherein the inert inorganic materials are selected from talc, titanium dioxide, zinc oxide, kaolin, zeolites, SiO₂, silica gel, calcium phosphate, calcium carbonate.

6. A process as claimed in any one of claims 1 to 5, in which the weight ratios of active ingredient to inert carrier range from 1:10 to 10:1.

## Patentansprüche

1. Verfahren zur Herstellung von Co-Mahlmaterialien zur kosmetischen und dermatologischen Verwendung, umfassend Co-Vermahlen, ohne die Verwendung von Lösungsmitteln, (i) mindestens eines kosmetischen Wirkstoffs und (ii) eines Trägers, der fähig ist, die hydrophilen oder lipophilen Charakteristika des Systems oder seine Freisetzungs- oder Absorptionscharakteristika zu modifizieren, und/oder inerter anorganischer Materialien in homogen gespaltenen kristallinen oder amorphen Formen, wobei der kosmetische Wirkstoff aus Hydratisierungsmitteln, Nährstoffen, erweichenden Mitteln, Linderungsmitteln, Sonnenschutzmitteln, Anti-aging-Mitteln, Depigmentierungsmitteln, Enthaarungsmitteln, Farbstoffen, Pigmenten, Aromastoffen oder Duftstoffen ausgewählt ist.

2. Verfahren nach Anspruch 1, wobei mit dem Träger ein Wirkstoffingredienz co-vermahlen wird.

3. Verfahren nach Anspruch 2, wobei die Wirkstoffe fest sind oder durch Absorptions-, Einschluss- oder Mikroeinkapslungstechniken festgemacht wurden.

4. Verfahren nach Anspruch 1 oder 2, wobei die Träger hydrophile oder lipophile Polymere, ausgewählt aus Crospovidon, Pektin, Dextran, Polyvinylpyrrolidon, Cellulose und ihren Derivaten, Stärken und ihren Derivaten; Cyclodextrinen; Phospholipiden; Fettsäuren; Zuckern, sind.

5. Verfahren nach Anspruch 1, wobei die inerten anorganischen Materialen aus Talk, Titandioxid, Zinkoxid, Kaolin, Zeolithen, SiO₂, Silicagel, Calciumphosphat, Calciumcarbonat ausgewählt werden.

6. Verfahren nach einem der Ansprüche 1 bis 5, wobei die Gewichtsverhältnisse von Wirkstoffingredienz zu inertem Träger im Bereich von 1:10 bis 10:1 liegen.

## Revendications

1. Un procédé pour la préparation de produits co-broyés pour l'usage cosmétique et dermatologique, comprenant le co-broyage, sans utilisation de solvants, de (i) au moins un ingrédient cosmétique actif et (ii) un support capable de modifier les caractéristiques hydrophiles ou lipophiles du système ou ses caractéristiques de libération ou d'absorption, et/ou des matières minérales inertes, sous les formes amorphes ou cristallines divisées de façon homogène, ledit ingrédient cosmétique actif étant choisi parmi les agents hydratants, les nutriments, les émollients, les agents lénifiants, les agents de protection anti-solaire, les agents anti-vieillissement, les agents de dépigmentation, les agents dépilatoires, les colorants, les pigments, les arômes ou les parfums.

2. Un procédé tel que revendiqué dans la revendication 1, dans lequel un ingrédient actif est co-broyé avec un support.

3. Un procédé tel que revendiqué dans la revendication 2, dans lequel les ingrédients actifs sont solides ou ont été rendus solides par des techniques d'absorption, d'inclusion ou de micro-encapsulation.

4. Un procédé tel que revendiqué dans la revendication 1 ou 2, dans lequel les supports sont des polymères hydrophiles ou lipophiles choisis parmi la povidone réticulée, la pectine, le dextrane, la polyvinylpyrrolidone, la cellulose et ses dérivés, les amidons et leurs dérivés ; les cyclodextrines ; les phospholipides ; les acides gras ; les sucres.

5. Un procédé tel que revendiqué dans la revendication 1, dans lequel les matières minérales inertes sont choisies parmi le talc, le bioxyde de titane, l'oxyde de zinc, le kaolin, les zéolites, SiO₂, le gel de silice, le phosphate de calcium, le carbonate de calcium.

6. Un procédé tel que revendiqué dans l'une quelconque des revendications 1 à 5, dans lequel les rapports en poids de l'ingrédient actif au support inerte sont compris entre 1:10 et 10:1.
